# FASCICULE DE BREVET EUROPEEN

(11) **EP 2 776 827 B1**
(45) Date de publication et mention de la délivrance du brevet: **09.12.2020**
(21) Numéro de dépôt: 12795502.9
(22) Date de dépôt: 08.11.2012
(51) Int. Cl.: G01N 33/00

(54) **DISPOSITIF COMPACT DE DÉTECTION DE GAZ ET DE VAPEURS AVEC CHARGE DE GAZ TEST INTÉGRÉE**
KOMPAKTE VORRICHTUNG ZUM NACHWEIS VON GASEN UND DÄMPFEN MIT EINER INTEGRIERTEN PRÜFGASLADUNG
COMPACT DEVICE FOR DETECTING GASES AND VAPOURS COMPRISING AN INTEGRATED TEST GAS CHARGE

(30) Priorité: 08.11.2011 FR 1103386
(43) Date de publication de la demande: 17.09.2014
(73) Titulaire: Centrexpert SAS, 69320 Feyzin (FR)
(72) Inventeur: CONSTANT, Jean-Marie, F-69006 Lyon (FR); MAURICE, Thierry, F-43620 Saint Romain Lachalm (FR)
(74) Mandataire: Chevalier, Renaud Philippe
(86) Numéro de dépôt international: PCT/FR2012/052577
(87) Numéro de publication internationale: WO 2013/068695

(56) Documents cités:
- DE-A1- 19 708 052
- FR-A1- 2 583 138
- GB-A- 2 362 217
- GB-A- 2 369 888
- US-A- 4 352 099
- US-A1- 2003 150 252
- US-A1- 2010 326 165
- US-B1- 7 840 366

## Description

La présente invention concerne un dispositif de détection de gaz et de vapeurs, et en particulier de gaz et de vapeurs inflammables.

On connaît de tels dispositifs de détection de gaz et de vapeurs qui fonctionnent par analyse de l'atmosphère ambiante d'un local, par catalyse ou par infrarouge ou par d'autres systèmes.

Ces dispositifs de détection de gaz et de vapeurs peuvent consister :
- soit en des installations conçues pour être installées de manière permanente dans un local ou sur un équipement,
- soit en des appareils portables qui peuvent par exemple être aisément transportés d'un local à un autre. Leur conception est alors souvent compacte.

Dans ces dispositifs de détection de gaz et de vapeurs, l'unité de détection peut faire partie intégrante du dispositif de détection de gaz et de vapeurs qui comporte alors des éléments d'analyse, de mesure et de lecture de concentration de gaz.

Ou bien, dans d'autres formes de réalisation du dispositif de détection de gaz et de vapeurs, l'unité de détection peut être séparée des éléments d'analyse, de mesure et de lecture de concentration de gaz qui sont alors regroupés dans une unité de contrôle extérieure au dispositif de détection de gaz et de vapeurs.

Les unités de détection de ces dispositifs de détection de gaz et de vapeurs doivent être périodiquement testées selon les indications du fabricant du dispositif de détection de gaz et de vapeurs.

Ces indications de test sont variables d'un fabricant à un autre.

De plus, les unités de détection de tels dispositifs de détection de gaz et de vapeurs doivent être également périodiquement calibrées ou étalonnées, selon les indications du fabricant, afin que la détection de gaz et de vapeurs s'opère au niveau des seuils de concentration de gaz et de vapeurs dans l'atmosphère indiqués par le fabricant comme détectables par le dispositif de détection de gaz et de vapeurs. Cette périodicité de calibrage et d'étalonnage est aussi variable d'un fabricant à un autre.

Une obligation normative selon la norme européenne EN 1755 prévoit un test de fonctionnement de l'unité de détection à chaque démarrage du dispositif de détection de gaz et de vapeurs, lorsque celui-ci est embarqué sur un chariot de manutention ou un autre véhicule utilisé en zone potentiellement explosive.

Les tests et les calibrations s'opèrent généralement par l'injection d'un flux de gaz de concentration connue et sélectionné par le fabricant dans l'unité de détection et par la comparaison des mesures de concentration détectée dans l'unité de détection et la concentration connue du gaz injecté. Ce gaz de concentration connue est ci-après appelé « gaz test ».

Cette injection de gaz test dans l'unité de détection est communément réalisée avec une bouteille de gaz sous pression.

Lorsqu'un tel dispositif de détection de gaz et de vapeurs (dans par ex. GB 2 369 888 A, GB 2 362 217 A) est embarqué sur une machine ou lorsque son installation est rendue difficile par manque d'espace, ou bien encore lorsque le fonctionnement optimum de l'unité de détection nécessite que son positionnement soit déporté dans des emplacements peu aisément accessibles, cela nécessite de relier l'unité de détection à un moyen de contrôle des liaisons électriques et à un moyen de test ou de calibration avec un tuyau d'amenée du gaz test.

Dans cette configuration, le tuyau d'amenée du gaz test peut être percé ou bouché pour une raison quelconque et perturber, voire même empêcher la mesure du test ou de la calibration du dispositif de détection de gaz et de vapeurs.

Par ailleurs, les distances entre les moyens de test ou de calibration et l'unité de détection peuvent être variables selon le dispositif de détection de gaz et de vapeurs.

Aussi, pour obtenir un test ou une calibration valide, cela nécessite de disposer d'une quantité de gaz test suffisante et non perturbée par la charge d'air en tampon entre la charge de gaz test et l'unité de détection pour la mesure. En effet, lorsque la charge de gaz test est éloignée de l'unité de détection, il y a un tampon d'air entre la charge de gaz test et l'unité de détection qu'il convient de pousser et de sortir de l'environnement de l'unité de détection pour détecter le gaz test et donc pour tester ou calibrer le dispositif de détection de gaz et de vapeurs. Plus la distance est grande, plus le tampon d'air est important, plus il faut de temps pour l'expulser, et plus cela consomme de gaz test, et ainsi plus il est nécessaire d'attendre pour obtenir les résultats de mesure obtenus à partir de l'unité de détection.

La prise en compte du tampon d'air oblige ainsi à opérer avec une bouteille de gaz sous pression et/ou de volume important. La bouteille de gaz utilisée est alors généralement encombrante de par sa taille et aussi de par la présence d'accessoires obligatoires, tels qu'un manomètre et un détendeur.

La nécessité de devoir réaliser le test ou la calibration avec une bouteille de gaz comprimé peut amener à dissuader l'utilisateur du dispositif de détection de gaz et de vapeurs de respecter scrupuleusement les instructions du fabricant en ce qui concerne les opérations de test et de calibration dudit dispositif de détection. Cela augmente alors le risque d'avoir des instruments de sécurité défaillants (car insuffisamment testés ou calibrés), puisqu'il s'agit de dispositifs destinés à détecter des valeurs de concentration de gaz qui pourraient être combustibles et/ou dangereux dans des locaux.

De plus, les distances variables entre l'unité de détection et les moyens de test ou de calibration du dispositif de détection de gaz et de vapeurs peuvent perturber le signal électrique relatif à la mesure du test ou de la calibration.

La présente invention se propose de remédier à l'ensemble de ces inconvénients de moyens de test ou de calibration d'un dispositif de détection de gaz et de vapeurs, qui sont parfois amenés à utiliser une bouteille de gaz comprimé très encombrante et très lourde..

En effet, la présente invention propose un dispositif de détection de gaz et de vapeurs qui ne nécessite pas de bouteille de gaz comprimé externe au dispositif mais qui, au contraire, intègre sa propre charge de gaz test d'environ 3 à 6 cm³, et dont le volume total du dispositif est minimal, pouvant être de l'ordre de 250 à 550 cm³.

Le dispositif de détection de gaz et de vapeurs selon l'invention se caractérise en ce qu'il comprend : a l'intérieur d'un boîtier
- au moins une unité de détection de gaz et de vapeurs ;
- au moins un réservoir contenant au moins un gaz test ;
- au moins un moyen de libération du gaz test contenu dans le réservoir dans un espace déterminé du dispositif ;
- au moins une unité de commande agencée pour provoquer la libération d'une quantité déterminée de gaz test dans l'espace déterminé par ledit moyen de libération et pour procéder à une mesure du gaz test détecté par l'unité de détection dans ledit espace déterminé ;
l'espace déterminé dans lequel est libéré le gaz test se situant en regard de l'unité de détection, le dispositif étant configuré pour que la distance parcourue par le gaz test entre le réservoir configuré pour contenir le gaz test et l'unité de détection soit au maximum de 30 mm.

Avantageusement, l'unité de détection de gaz et de vapeurs consiste en un Pellistor.

De manière avantageuse, le dispositif selon l'invention se présente sous la forme d'un boîtier. De manière préférée, le boîtier du dispositif selon l'invention a un volume compris entre 100 et 800 cm³, de préférence entre 250 et 600 cm³, encore plus préférentiellement entre 300 et 550 cm³.

Par exemple, le boîtier peut présenter une forme générale cylindrique et être équipé à une première extrémité d'un couvercle et à la seconde extrémité d'une embase.

Le boîtier du dispositif peut être réalisé en un matériau plastique par injection permettant l'obtention de logements parfaitement positionnés des divers éléments précités du dispositif, et leur montage sans difficulté.

Dans un autre mode de réalisation de l'invention, le boîtier peut être réalisé en métal par usinage.

Le boîtier peut en outre comporter au moins un joint pour assurer son étanchéité.

L'embase peut être destinée à supporter l'un des éléments détaillés ci-dessus que comprend le dispositif selon l'invention.

Avantageusement, le boîtier présente un corps de forme générale cylindrique dont le diamètre est compris entre 40 et 100 mm, de préférence entre 60 et 80 mm et la longueur est comprise entre 70 et 200 mm, de préférence entre 90 et 110 mm. Bien entendu, d'autres formes de réalisation du boîtier sont envisageables dans le cadre de la présente invention et sont parfaitement à la portée de l'homme du métier.

De manière avantageuse, le couvercle et/ou l'embase peuvent être vissés au corps du boîtier. Bien entendu, tout autre mode de fixation du couvercle et/ou de l'embase sur le corps à la portée de l'homme du métier peut être envisagé et entre dans le cadre de la présente invention.

De plus, de manière avantageuse, le dispositif selon l'invention peut être configuré pour que la distance parcourue par le gaz test entre le réservoir contenant le gaz test et l'unité de détection soit de préférence au maximum de 20 mm, et encore plus préférentiellement au maximum de 10 mm. Cela permet des injections de gaz test de l'ordre de 30 millisecondes, de préférence de l'ordre de 10 millisecondes, et encore plus préférentiellement de l'ordre de 3 millisecondes, sous une pression pouvant être comprise entre 1,5 et 7 bars.

Il est à noter qu'avec une distance maximale de l'ordre de 20 mm, le tampon d'air évoqué ci-dessus est expulsé en moins d'une milliseconde.

Du fait de la très courte distance parcourue par le gaz test entre le réservoir et l'unité de détection, la charge de gaz test nécessaire pour la réalisation du test de fonctionnement du dispositif selon l'invention est très faible comparée à celle d'autres dispositifs de détection évoqués ci-dessus, et par conséquent, la libération du gaz test nécessite aussi une très faible énergie.

En outre, du fait de cette économie de consommation de gaz test pour réaliser le test de fonctionnement du dispositif de détection selon l'invention, cela présente l'avantage que la durée de vie de la charge de gaz test est plus longue que pour d'autres dispositifs de détection de l'état de la technique, ainsi que celle de la source d'énergie nécessaire à la libération du gaz test.

Le gaz test contenu dans le réservoir peut être choisi parmi n'importe quel gaz inflammable brûlant à une température inférieure à 400°C. De préférence, il s'agit d'isobutane ou de propane.

Dans un mode de réalisation de l'invention, le volume du réservoir contenant au moins un gaz test est compris entre 2 et 10 cm³, de préférence entre 2,5 et 6 cm³, avantageusement de l'ordre de 3 cm³.

Le réservoir est avantageusement réalisé en un matériau rigide. Le gaz test contenu dans le réservoir peut se trouver au moins partiellement à l'état liquide, de manière à ce que le débit de gaz test soit sensiblement constant lors de sa libération hors du réservoir.

Un logement peut être prévu dans le dispositif selon l'invention pour y loger le réservoir de gaz test. Ainsi, cela présente l'avantage que le réservoir de gaz étant amovible, il peut être retiré du dispositif selon l'invention et aisément être rechargé en gaz test, si le niveau de gaz test est insuffisant pour réaliser les tests de fonctionnement dudit dispositif.

Le réservoir contenant au moins un gaz test peut consister en le réservoir d'un briquet, et par exemple un briquet jetable.

Dans d'autres modes de réalisation, le réservoir peut consister en tout container étanche, par exemple réalisé en un matériau rigide, équipé d'au moins un premier orifice agencé pour la libération du gaz test hors du réservoir, et éventuellement d'au moins un deuxième orifice pour recharger le réservoir en gaz test.

Selon un mode de réalisation de l'invention, lorsque le gaz test est contenu dans le réservoir d'un briquet (par exemple un briquet jetable) auquel on a retiré la molette, le moyen de libération du gaz test dans l'espace déterminé peut consister en l'association d'un électroaimant, de la tige de clapet et de la gâchette du briquet.

La tige de clapet qui assure l'étanchéité du réservoir du briquet est soulevée par la gâchette de manière à libérer le gaz test hors du réservoir du briquet dans l'espace déterminé, lorsque la gâchette est elle-même actionnée par l'électroaimant.

Dans un autre mode de réalisation de l'invention assez proche de ce dernier mode de réalisation, le moyen de libération du gaz test peut consister en l'association d'un électroaimant, de la tige de clapet et de la gâchette d'un briquet et d'un tuyau d'amenée du gaz test, ledit tuyau d'amenée du gaz test étant agencé pour amener le gaz test de la tige de clapet du briquet jusqu'à l'unité de détection du dispositif.

Dans ce mode de réalisation de l'invention, lorsque l'électroaimant est déclenché par une séquence ordonnée par l'unité de commande, il appuie sur la gâchette qui lève la tige de clapet. Une quantité déterminée du gaz test contenu dans le réservoir s'échappe dans le tube d'amenée du gaz test jusqu'à l'unité de détection du dispositif qui enregistre la valeur détectée et la transmet à ladite unité de commande.

Le tuyau d'amenée du gaz peut éventuellement être équipé à son extrémité du côté du réservoir du briquet d'un embout femelle, de préférence en un matériau plastique, et qui est configuré pour que la tige de clapet du briquet y soit introduite.

Dans un autre mode de réalisation de l'invention, lorsque le réservoir de gaz test est un réservoir de briquet (par exemple un briquet jetable) auquel on a retiré la molette, le moyen de libération de gaz test peut consister en l'association de la gâchette et la tige de clapet du briquet et d'une électrovanne.

Pour ce faire, en position hors de toute libération de gaz test :
- la gâchette du briquet peut être disposée en appui sur une embase que comporte le dispositif de manière à ce que la tige de clapet soit soulevée, et
- l'électrovanne est en position fermée et bloque la libération du gaz test au travers la tige de clapet.

Lorsqu'une charge de gaz test doit être libérée dans l'espace déterminé, l'électrovanne est ouverte au moyen de l'unité de commande et laisse sortir le gaz test du réservoir du briquet au travers la tige de clapet. Le gaz test est ainsi libéré dans l'espace déterminé.

Selon un autre mode de réalisation de l'invention, le réservoir de gaz test se présente sous la forme d'un container équipé d'un orifice. Le moyen de libération du gaz test consiste en une électrovanne, ladite électrovanne étant agencée pour obstruer l'orifice du container lorsqu'elle est en position fermée. Ainsi, en position hors de toute libération de gaz test, l'orifice du container est obstrué par l'électrovanne qui est en position fermée. A l'ouverture de l'électrovanne, le gaz test est libéré dans l'espace déterminé en passant au travers de l'orifice du container.

De plus, dans ce mode de réalisation de l'invention, il peut être envisagé de disposer le container sur une embase que comporte le dispositif, de manière à ce que l'orifice du réservoir soit en regard d'un trou qui traverse de part en part ladite embase et l'électrovanne étant disposée de manière à être en regard avec le trou de l'embase. Ainsi, l'électrovanne en position fermée bloque la libération du gaz test qui est présent aussi dans le trou de l'embase. Et lorsque l'électrovanne est ouverte au moyen de l'unité de commande du dispositif, le gaz test est libéré dans l'espace déterminé qui se situe en regard de l'unité de détection.

Selon un autre mode de réalisation de l'invention, le réservoir de gaz test présente la forme d'un corps s'étendant selon une direction longitudinale, le corps est pourvu à une première extrémité d'un orifice agencé pour libérer le gaz test dans l'espace déterminé. La première extrémité est maintenue en appui sur une embase que comporte le dispositif de manière à ce que l'orifice soit obstrué par l'embase. Un électroaimant est agencé à proximité de la seconde extrémité du corps pour le faire pivoter autour d'un axe de manière à ce que l'orifice soit dégagé de l'embase et que du gaz test soit libéré hors du réservoir. Le corps du réservoir agit alors comme un bras de levier. L'axe de pivotement peut notamment être parallèle au plan défini par l'embase.

En particulier, dans le cas de l'utilisation d'un réservoir formé par un réservoir de briquet (par exemple un briquet jetable), il est possible de faire pivoter le corps du réservoir autour d'un axe logé dans le pivot utilisé pour monter une molette.

Dans un mode de réalisation de l'invention, le dispositif comprend en outre au moins un premier moyen d'alimentation de l'unité de commande. Il peut s'agir d'une batterie ou d'une pile qui est intégrée dans le dispositif selon l'invention qui se présente, par exemple, sous la forme d'un boîtier. Ou bien, le premier moyen d'alimentation est extérieur au dispositif selon l'invention : il peut être commandé par une unité de contrôle extérieure au dispositif selon l'invention.

Selon un autre mode de réalisation de l'invention, le dispositif comprend au moins un deuxième moyen d'alimentation du moyen de libération du gaz test dans l'espace déterminé. Il peut s'agir d'une batterie ou d'une pile qui est intégrée dans le dispositif selon l'invention qui se présente, par exemple, sous la forme d'un boîtier. Ou bien, le deuxième moyen d'alimentation est extérieur au dispositif selon l'invention : il peut être commandé par une unité de contrôle extérieure au dispositif selon l'invention.

Selon un mode de réalisation de l'invention, le premier moyen d'alimentation de l'unité de commande et le deuxième moyen d'alimentation du moyen de libération du gaz hors du réservoir consistent en un unique moyen d'alimentation qui peut consister avantageusement en une batterie ou en une pile.

De manière préférée, l'unité de commande peut consister en une carte électronique. La carte électronique peut comporter des moyens de communication sans fil, en particulier constitués par un processeur de communication sans fil (par exemple de type ZigBee ®).

Dans un mode de réalisation de l'invention, le dispositif est destiné à interagir avec une unité de contrôle par l'intermédiaire des moyens de communication sans fil.

L'unité de contrôle traite les informations recueillies par l'unité de commande.

Ainsi, les informations sont basiquement lues au niveau de l'unité de commande et envoyées vers l'unité de contrôle qui peut présenter la forme d'une tablette tactile, utilisant par exemple un système d'exploitation Androïd, et qui décrypte les valeurs qui sont analysées par le logiciel dont est équipé l'unité de contrôle.

Dans un mode de réalisation de l'invention, le moyen de libération est connecté à un processeur de l'unité de contrôle par une interface sans fil.

Dans un mode de réalisation de l'invention, l'unité de détection envoie un signal non traité au processeur de l'unité de contrôle extérieur par une interface sans fil.

La présente invention concerne aussi un procédé de test de fonctionnement d'un dispositif de détection de gaz et de vapeurs tel que décrit ci-dessus, ledit procédé se caractérisant en ce qu'il comprend les étapes suivantes :
a) on dispose d'un dispositif de détection de gaz et de vapeurs tel que décrit ci-dessus ;
b) on libère une quantité déterminée de gaz test hors du réservoir de gaz test du dispositif ;
c) on collecte des données de mesure de concentration de gaz test dans l'espace déterminé ;
d) on détermine sur la base des données de mesure collectée si la concentration de gaz test dépasse un premier seuil haut de concentration, et si la concentration descend ensuite en dessous d'un deuxième seuil bas de concentration, inférieur au premier seuil haut ;
e) on détermine un état de fonctionnement dudit dispositif de détection de gaz et de vapeurs en fonction de la détermination de l'atteinte du premier et du deuxième seuil de concentration.

De manière avantageuse, on préchauffe l'unité de détection jusqu'à ce qu'elle atteigne une température déterminée avant de réaliser l'étape b).

Le temps de préchauffage de l'unité de détection est variable selon différents paramètres que sont notamment la température extérieure ambiante.

Le préchauffage de l'unité de détection peut être commandé au moyen de l'unité de commande du dispositif.

Si l'unité de détection consiste en un Pellistor, au cours du préchauffage, la tension du Pellistor va diminuer avec le temps au fur et à mesure de la montée en température. La tension du Pellistor est avantageusement mesurée toutes les 250 millisecondes jusqu'à la stabilité de la courbe de la tension du Pellistor en fonction du temps. Cette phase de préchauffage est de l'ordre de 5 à 10 secondes, et permet de vérifier que le Pellistor chauffe correctement.

Il peut être prévu que les données analogiques du Pellistor soient envoyées par signal ZigBee ® à partir de l'unité de commande à l'unité de contrôle qui les traite numériquement.

Si la courbe de la tension ne se stabilise pas au terme d'une durée déterminée, de préférence d'environ 40 secondes, le dispositif de détection de gaz et de vapeurs selon l'invention est déclaré défectueux.

Lorsque la stabilité de la courbe de la tension du Pellistor en fonction du temps est obtenue, l'unité de commande enregistre le niveau de celle-ci qui détermine le zéro (phase de calage) et provoque la libération d'une quantité déterminée de gaz test dans l'espace déterminé.

Il est particulièrement avantageux de libérer le gaz test juste au moment où la courbe de la tension du Pellistor en fonction du temps devient stable ; car il s'agit du moment ou l'efficacité du Pellistor est maximale pour détecter le gaz test dans l'espace déterminé.

Les molécules de gaz test sont immédiatement brûlées par le Pellistor du fait de la proximité et la concentration maximales du gaz test dans l'espace déterminé qui est en regard du Pellistor. La détection de gaz test est signalée à l'unité de contrôle par l'intermédiaire de l'unité de commande.

L'unité de contrôle analyse que la concentration en gaz test mesurée, et en particulier :
1) qu'un un premier seuil haut de concentration est franchi, et qui correspond avantageusement à 25% de la Limite Inférieure d'Explosivité du gaz test (par exemple de l'isobutane) ; ce qui indique que le « Pellistor » fonctionne correctement, et
2) qu'elle retombe au-dessous d'un deuxième seuil bas de concentration, et qui correspond avantageusement à 10% de la Limite Inférieure d'Explosivité du gaz test, ce qui indique que l'atmosphère environnante est sûre.

Si ces deux seuils sont franchis, l'unité de contrôle envoie alors un signal vers l'unité de commande du dispositif pour mettre le dispositif de détection de gaz et de vapeurs en état de fonctionnement.

En cours de fonctionnement du dispositif de détection de gaz et de vapeurs selon l'invention, une détection à 10% de la Limite Inférieure d'Explosivité alarme, et à 25% de la Limite Inférieure d'Explosivité l'unité de contrôle envoit un signal de coupure immédiate au dispositif de détection de gaz et de vapeurs et à l'équipement qu'il asservit.

De toute façon, l'invention sera mieux comprise à l'aide de la description qui suit, en référence au dessin schématique annexé, représentant à titre d'exemple, deux formes d'exécution du dispositif de détection de gaz et de vapeurs selon l'invention.
La figure 1 représente en perspective et de manière éclatée un dispositif de détection de gaz et de vapeurs selon un premier mode de réalisation de l'invention.
La figure 2 représente en perspective et de manière éclatée une partie du dispositif de détection de gaz et de vapeurs selon un deuxième mode de réalisation de l'invention.
La figure 3 représente en perspective la partie du dispositif de détection de gaz et de vapeurs représenté à la figure 2 en mode assemblé.

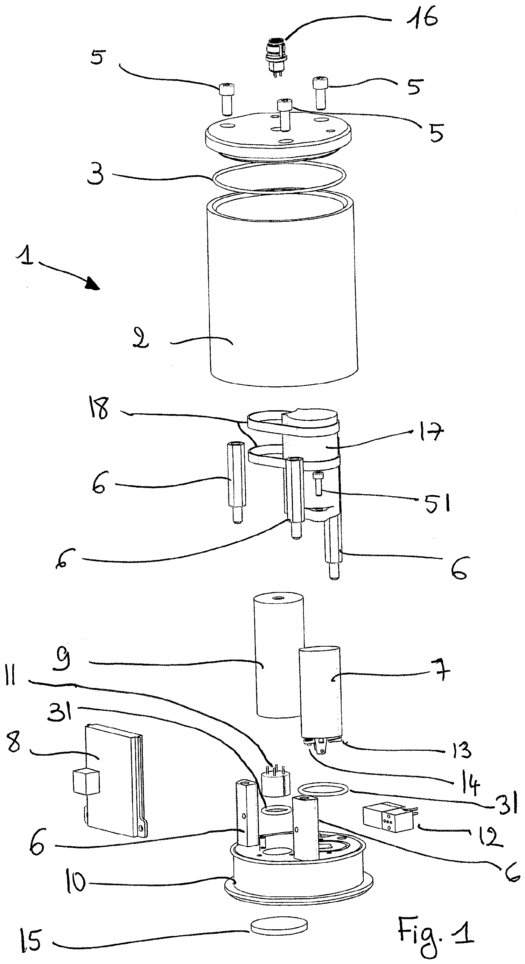

En référence à la figure 1, le dispositif 1 de détection de gaz et de vapeurs comprend un corps 2 de forme cylindrique équipé d'un couvercle 4 qui est vissé dessus au moyen de trois vis 5. Un joint 3 assure l'étanchéité entre le couvercle 4 et le corps 2.

Le dispositif 1 comprend en outre une embase 10, un briquet 7 avec une gâchette 13 et une tige de clapet 14.

Le réservoir du briquet 7 contient un gaz test qui est du propane. Un réservoir 17 est prévu pour y disposer à l'intérieur le briquet 7. A l'intérieur du corps 2 du dispositif 1 sont aussi disposés :
- une carte électronique 8 (à savoir l'unité de commande du dispositif 1),
- une électrovanne 12 qui contribue en association avec la tige de clapet 13 et la gâchette 14 du briquet 7 à la libération de gaz test,
- une batterie 9 destinée à alimenter l'électrovanne 12 et la carte électronique 8.

L'unité de détection du dispositif 1 est un pellistor 11.

Le dispositif 1 comprend en outre un connecteur d'alimentation 16 dans le cas où l'on souhaite s'affranchir de la batterie 9.

Des moyens d'assemblage 18 de la batterie 9 et du réservoir 17 entre eux sont prévus. Il s'agit d'élastiques en caoutchouc.

Le dispositif 1 comprend aussi :
- une rondelle de protection 15 ;
- des moyens de fixation 6 du corps 2 sur l'embase 10 ;
- deux joints 31 qui sont disposés sur l'embase 10 ;
- une vis 51 permettant de visser le réservoir 17 sur l'embase 10.

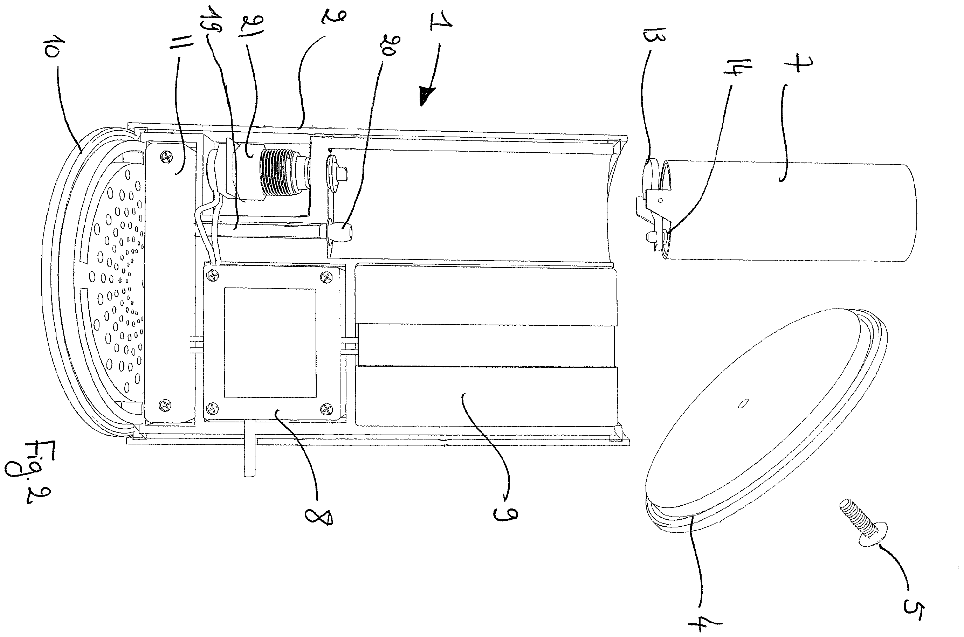

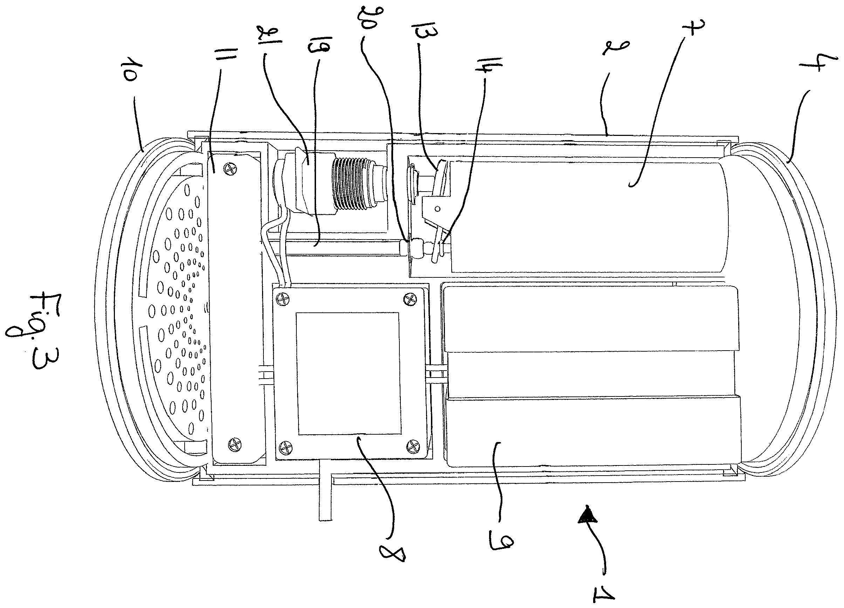

Pour le dispositif 1 représenté sur les figures 2 et 3, le moyen de libération du gaz test consiste en l'association d'un embout femelle 20, d'un tuyau d'amenée de gaz test 19, d'un électroaimant 21, de la gâchette13 et la tige de clapet 14 du briquet 7. L'unité de détection est un Pellistor 11 et l'unité de commande est une carte électronique 8. Le couvercle 4 est vissé sur le corps 2 du dispositif 1 au moyen d'une vis 5. Le dispositif 1 est pourvu d'une embase 10. Le moyen d'alimentation de la carte électronique 8 et de l'électroaimant 21 est une batterie 9.

Une unité de contrôle extérieure au dispositif 1 peut être prévue. Elle n'a pas été représentée sur les figures 1 à 3.

Bien entendu, l'exemple de forme d'exécution évoqué ci-dessus ne présente aucun caractère limitatif et d'autres améliorations et détails, notamment d'autres procédés de fabrication que ceux évoqués ci-dessus peuvent être envisagés, peuvent être apportés au dispositif de détection de gaz et de vapeurs selon l'invention, sans pour autant sortir du cadre de l'invention défini par les revendications annexées.

## Revendications

1. Dispositif (1) de détection de gaz et de vapeurs se présentant sous la forme d'un boîtier qui comprend en son sein au moins une unité de détection de gaz et de vapeurs (11),
caractérisé en que ledit boîtier comprend en outre, en son sein:
- au moins un réservoir (7) contenant au moins un gaz test ;
- au moins un moyen de libération du gaz test (12,13,14,19,21) contenu dans le réservoir (7) dans un espace déterminé du dispositif (1) ;
- au moins une unité de commande (8) agencée pour provoquer la libération d'une quantité déterminée de gaz test dans l'espace déterminé par ledit moyen de libération (12,13,14,19,21) et pour procéder à une mesure du gaz test détecté par l'unité de détection (11) dans ledit espace déterminé ;
l'espace déterminé dans lequel est libéré le gaz test se situant en regard de l'unité de détection (11), le dispositif (1) étant configuré pour que la distance parcourue par le gaz test entre le réservoir (7) configuré pour contenir le gaz test et l'unité de détection (11) soit au maximum de 30 mm.

2. Dispositif (1) selon la revendication 1, **caractérisé en ce que** l'unité de détection de gaz et de vapeurs consiste en un Pellistor (11).

3. Dispositif (1) selon l'une quelconque des revendications précédentes, **caractérisé en ce que** le dispositif (1) se présente sous la forme d'un boîtier (2) de volume compris entre 100 et 800 cm³.

4. Dispositif (1) selon l'une quelconque des revendications précédentes, **caractérisé en ce que** le volume du réservoir (7) configuré pour contenir au moins un gaz test est compris entre 2 et 10 cm³.

5. Dispositif (1) selon l'une quelconque des revendications précédentes, **caractérisé en ce que** le réservoir configuré pour contenir au moins un gaz test consiste en le réservoir d'un briquet (7).

6. Dispositif (1) selon la revendication précédente, **caractérisé en ce que** le moyen de libération du gaz test dans l'espace déterminé consiste en l'association d'un électroaimant (21), de la tige de clapet (14) et de la gâchette (13) du briquet (7).

7. Dispositif (1) selon la revendication 5, **caractérisé en ce que** le moyen de libération du gaz test consiste en l'association d'un électroaimant (21), de la tige de clapet (14) et de la gâchette (13) d'un briquet (7) et d'un tuyau d'amenée du gaz test (19), ledit tuyau d'amenée du gaz test (19) étant agencé pour amener le gaz test du réservoir du briquet (7) jusqu'à l'unité de détection du dispositif (11).

8. Dispositif (1) selon la revendication 5, **caractérisé en ce que** le moyen de libération de gaz test consiste en l'association de la gâchette (13) et de la tige de clapet (17) du briquet (7) et d'une électrovanne (12).

9. Dispositif (1) selon la revendication 1, **caractérisé en ce que** le réservoir (7) de gaz test présente la forme d'un corps s'étendant selon une direction longitudinale, le corps étant pourvu à une première extrémité d'un orifice agencé pour libérer le gaz test dans l'espace déterminé, la première extrémité étant maintenue en appui sur une embase que comporte le dispositif de manière à ce que l'orifice soit obstrué par l'embase et un électroaimant étant agencé à proximité de la seconde extrémité du corps pour le faire pivoter autour d'un axe de manière à ce que l'orifice soit dégagé de l'embase et que du gaz test soit libéré hors du réservoir.

10. Dispositif (1) selon l'une quelconque des revendications précédentes, **caractérisé en ce que** l'unité de commande consiste en une carte électronique (8).

11. Dispositif (1) selon l'une quelconque des revendications précédentes, caractérisé en ce le dispositif (1) comprend en outre au moins un premier moyen d'alimentation de l'unité de commande (8) et/ou au moins un deuxième moyen d'alimentation du moyen de libération (12,13,14,19,21) du gaz test dans l'espace déterminé.

12. Dispositif (1) selon la revendication précédente, **caractérisé en ce que** le premier moyen d'alimentation et le deuxième moyen d'alimentation consistent en un unique moyen d'alimentation (9).

13. Procédé de test de fonctionnement d'un dispositif (1) de détection de gaz et de vapeurs selon l'une quelconque des revendications précédentes, **caractérisé en ce qu'**il comprend les étapes suivantes :
a) on dispose d'un dispositif (1) de détection de gaz et de vapeurs selon l'une quelconque des revendications précédentes ;
b) on libère une quantité déterminée de gaz test hors du réservoir (7) de gaz test du dispositif (1);
c) on collecte des données de mesure de concentration de gaz test dans l'espace déterminé ;
d) on détermine sur la base des données de mesure collectée si la concentration de gaz test dépasse un premier seuil haut de concentration, et si la concentration descend ensuite en dessous d'un deuxième seuil bas de concentration, inférieur au premier seuil haut ;
e) on détermine un état de fonctionnement dudit dispositif (1) de détection de gaz et de vapeurs en fonction de la détermination de l'atteinte du premier et du deuxième seuil de concentration.

14. Procédé selon la revendication précédente, caractérisé en qu'on préchauffe l'unité de détection (11) du dispositif (1) jusqu'à ce qu'elle atteigne une température déterminée avant de réaliser l'étape b).

## Patentansprüche

1. Vorrichtung (1) zum Nachweis von Gasen und Dämpfen, die sich in Form eines Gehäuses darstellt, das in sich mindestens eine Einheit zum Nachweis von Gasen und Dämpfen (11) umfasst, **dadurch gekennzeichnet, dass** das Gehäuse des Weiteren in sich umfasst:
- mindestens einen Behälter (7), der mindestens ein Prüfgas enthält;
- mindestens ein Mittel zur Freisetzung des Prüfgases (12, 13, 14, 19, 21), das in dem Behälter (7) in einem bestimmten Raum der Vorrichtung (1) enthalten ist;
- mindestens eine Steuereinheit (8), die angeordnet ist, um die Freisetzung einer bestimmten Menge an Prüfgas in dem bestimmten Raum durch das Freisetzungsmittel (12, 13, 14, 19, 21) zu bewirken und um eine Messung des von der Nachweiseinheit (11) nachgewiesenen Prüfgases in dem bestimmten Raum vorzunehmen;
wobei sich der bestimmte Raum, in dem das Prüfgas freigesetzt wird, gegenüber der Nachweiseinheit (11) befindet, wobei die Vorrichtung (1) so ausgestaltet ist, dass der Abstand, der von dem Prüfgas zwischen dem Behälter (7), der ausgestaltet ist, das Prüfgas zu enthalten, und der Nachweiseinheit (11) zurückgelegt wird, höchstens 30 mm beträgt.

2. Vorrichtung (1) nach Anspruch 1, **dadurch gekennzeichnet, dass** die Einheit zum Nachweis von Gasen und Dämpfen aus einem Pellistor (11) besteht.

3. Vorrichtung (1) nach einem der vorstehenden Ansprüche, **dadurch gekennzeichnet, dass** sich die Vorrichtung (1) in Form eines Gehäuses (2) mit einem Volumen darstellt, das zwischen 100 und 800 cm³ beträgt.

4. Vorrichtung (1) nach einem der vorstehenden Ansprüche, **dadurch gekennzeichnet, dass** das Volumen des Behälters (7), der ausgestaltet ist, mindestens ein Prüfgas zu enthalten, zwischen 2 und 10 cm³ beträgt.

5. Vorrichtung (1) nach einem der vorstehenden Ansprüche, **dadurch gekennzeichnet, dass** der Behälter, der ausgestaltet ist, mindestens ein Prüfgas zu enthalten, aus dem Behälter eines Feuerzeugs (7) besteht.

6. Vorrichtung (1) nach dem vorstehenden Anspruch, **dadurch gekennzeichnet, dass** das Mittel zur Freisetzung des Prüfgases in dem bestimmten Raum aus der Verbindung eines Elektromagneten (21), der Kegelstange (14) und des Abzugs (13) des Feuerzeugs (7) besteht.

7. Vorrichtung (1) nach Anspruch 5, **dadurch gekennzeichnet, dass** das Mittel zur Freisetzung des Prüfgases aus der Verbindung eines Elektromagneten (21), der Kegelstange (14) und des Abzugs (13) eines Feuerzeugs (7) und einer Zuleitung des Prüfgases (19) besteht, wobei die Zuleitung des Prüfgases (19) angeordnet ist, um das Prüfgas von dem Behälter des Feuerzeugs (7) bis zu der Nachweiseinheit der Vorrichtung (11) zuzuleiten.

8. Vorrichtung (1) nach Anspruch 5, **dadurch gekennzeichnet, dass** das Mittel zur Freisetzung des Prüfgases aus der Verbindung des Abzugs (13) und der Kegelstange (17) des Feuerzeugs (7) und eines Magnetventils (12) besteht.

9. Vorrichtung (1) nach Anspruch 1, **dadurch gekennzeichnet, dass** sich der Behälter (7) des Prüfgases in Form eines Körpers darstellt, der sich gemäß einer Längsrichtung erstreckt, wobei der Körper an einer ersten Extremität mit einer Öffnung versehen ist, die angeordnet ist, um das Prüfgas in dem bestimmten Raum freizusetzen, wobei die erste Extremität auf einen Sockel gedrückt gehalten wird, den die Vorrichtung umfasst, sodass die Öffnung von dem Sockel blockiert wird, und wobei ein Elektromagnet in der Nähe der zweiten Extremität des Körpers angeordnet ist, um ihn um eine Achse schwenken zu lassen, sodass die Öffnung von dem Sockel freigelegt wird und Prüfgas aus dem Behälter freigesetzt wird.

10. Vorrichtung (1) nach einem der vorstehenden Ansprüche, **dadurch gekennzeichnet, dass** die Steuereinheit aus einer elektronischen Platine (8) besteht.

11. Vorrichtung (1) nach einem der vorstehenden Ansprüche, **dadurch gekennzeichnet, dass** die Vorrichtung (1) des Weiteren mindestens ein erstes Versorgungsmittel der Steuereinheit (8) und/oder mindestens ein zweites Versorgungsmittel des Mittels zur Freisetzung (12, 13, 14, 19, 21) des Prüfgases in dem bestimmten Raum umfasst.

12. Vorrichtung (1) nach dem vorstehenden Anspruch, **dadurch gekennzeichnet, dass** das erste Versorgungsmittel und das zweite Versorgungsmittel aus einem einzigen Versorgungsmittel (9) bestehen.

13. Verfahren zur Funktionsprüfung einer Vorrichtung (1) zum Nachweis von Gasen und Dämpfen nach einem der vorstehenden Ansprüche, **dadurch gekennzeichnet, dass** es folgende Schritte umfasst:
a) Bereitstellen einer Vorrichtung (1) zum Nachweis von Gasen und Dämpfen nach einem der vorstehenden Ansprüche;
b) Freisetzen einer bestimmten Menge an Prüfgas aus dem Behälter (7) des Prüfgases der Vorrichtung (1);
c) Sammeln von Messdaten einer Prüfgaskonzentration in dem bestimmten Raum;
d) Bestimmen anhand der gesammelten Messdaten, ob die Prüfgaskonzentration eine erste Konzentrationsobergrenze übersteigt und ob die Konzentration danach unter eine zweite Konzentrationsuntergrenze abfällt, die niedriger ist als die erste Obergrenze;
e) Bestimmen eines Funktionszustands der Vorrichtung (1) zum Nachweis von Gasen und Dämpfen in Abhängigkeit von der Bestimmung des Erreichens der ersten und der zweiten Konzentrationsgrenze.

14. Verfahren nach dem vorstehenden Anspruch, **dadurch gekennzeichnet, dass** die Nachweiseinheit (11) der Vorrichtung (1) vorerwärmt wird, bis sie eine bestimmte Temperatur erreicht hat, bevor Schritt b) ausgeführt wird.

## Claims

1. A device (1) for detecting gases and vapors is shaped as a casing which comprises within it at least one gases and vapors detection unit (11), **characterized in that** said casing further comprises, within it:
- at least one tank (7) containing at least one test gas;
- at least one means for releasing the test gas (12,13,14,19,21) contained in the tank (7) into a determined space of the device (1);
- at least one control unit (8) arranged to cause the release of a determined quantity of test gas into the space determined by the said release means (12,13,14,19,21) and to carry out a measurement of the test gas detected by the detection unit (11) in said determined space;
the determined space in which the test gas is released, is located opposite to the detection unit (11), the device (1) being configured so that the distance travelled by the test gas between the tank (7) configured to contain the test gas and the detection unit (11) is at most 30mm.

2. The device (1) according to claim 1, **characterized in that** the gases and vapors detection unit consists of a Pellistor (11).

3. The device (1) according to any one of the preceding claims, **characterized in that** the device (1) is shaped as a casing (2) of a volume comprised between 100 and 800 cm³.

4. The device (1) according to any one of the preceding claims, **characterized in that** the volume of the tank (7) configured to contain at least one test gas is comprised between 2 and 10cm³.

5. The device (1) according to any one of the preceding claims, **characterized in that** the tank configured to contain at least one test gas consists of the tank of a lighter (7).

6. The device (1) according to the preceding claim, **characterized in that** the means for releasing the test gas into the determined space consists of the combination of an electromagnet (21), the valve stem (14) and the trigger (13) of the lighter (7).

7. The device (1) according to claim 5, **characterized in that** the means for releasing the test gas consists of the combination of an electromagnet (21), the valve stem (14) and the trigger (13) of a lighter (7) and a test gas supply pipe (19), said test gas supply pipe (19) being arranged to supply the test gas from the lighter tank (7) to the detection unit of the device (11).

8. The device (1) according to claim 5, **characterized in that** the means for releasing the test gas consists of the combination of the trigger (13) and the valve stem (17) of the lighter (7) and a solenoid valve (12).

9. The device (1) according to claim 1, **characterized in that** the test gas tank (7) has the shape of a body extending according to a longitudinal direction, the body being provided at a first end of an orifice arranged to release the test gas in the determined space, the first end being held in abutment on a base which the device includes so that the orifice is obstructed by the base and an electromagnet being arranged near the second end of the body to pivot it about an axis so that the orifice is clear from the base and that a test gas is released outside the tank.

10. The device (1) according to any one of the preceding claims, **characterized in that** the control unit consists of an electronic card (8).

11. The device (1) according to any one of the preceding claims, **characterized in that** the device (1) further comprises at least one first means for supplying the control unit (8) and/or at least one second means for supplying the means for releasing (12, 13, 14, 19, 21) the test gas into the determined space.

12. The device (1) according to the preceding claim, **characterized in that** the first supply means and the second supply means consist of a single supply means (9).

13. A method for testing the operation of a device (1) for detecting gases and vapors according to any one of the preceding claims, **characterized in that** it comprises the following steps:
a) disposing a device (1) for detecting gases and vapors according to any one of the preceding claims;
b) releasing a determined quantity of test gas outside the test gas tank (7) of the device (1);
c) collecting data of the concentration measurement of the test gas in the determined space;
d) determining on the basis of the collected measurement data, whether the concentration of the test gas exceeds a first high concentration threshold, and whether the concentration subsequently falls below a second low concentration threshold, lower than the first high threshold;
e) determining an operating state of said device (1) for detecting gases and vapors depending on the determination of whether the first and second concentration thresholds have been reached.

14. The method according to the preceding claim, **characterized in that** the detection unit (11) of the device (1) is preheated until it reaches a predetermined temperature before carrying out step b).
